Europäisches Patentamt

(19) European Patent Office                    (11) Publication number: **0 027 347**

Office européen des brevets                                                **B1**

(12)                    **EUROPEAN PATENT SPECIFICATION**

(45) Date of publication of patent specification: **07.05.86**      (51) Int. Cl.⁴: **A 61 K 39/12,** A 61 K 39/215,
                                                                **C 12 N 7/08**
(21) Application number: **80303533.6**

(22) Date of filing: **08.10.80**

(54) Feline infectious peritonitis virus, its preparation and vaccines containing it.

(30) Priority: **16.10.79 US 85435**

(43) Date of publication of application:
**22.04.81 Bulletin 81/16**

(45) Publication of the grant of the patent:
**07.05.86 Bulletin 86/19**

(72) Inventor: **Davis, Eldon**
**7221 South Street**
**Lincoln, Nebraska 68520 (US)**

(84) Designated Contracting States:
**AT BE CH DE FR GB LI LU NL SE**

(73) Proprietor: **NORDEN LABORATORIES, INC.**
**601 W. Cornhusker Highway**
**Lincoln, Nebraska 68501 (US)**

(56) References cited:
**EP-A-0 011 864**
**EP-A-0 011 865**
**GB-A-1 177 635**
**GB-A-1 286 250**
**GB-A-1 471 262**
**GB-A-1 492 930**
**US-A-4 195 130**

(74) Representative: **Johnston, Walter Paul**
**P.O. Box 39**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to feline infectious peritonitis (FIP) virus, its preparation and to vaccines containing it.

Feline infectious peritonitis (FIP) is a disease of both domestic and wild cats characterized by progressive debilitation and, in the "wet" or effusive form, a fibrinous peritoneal exudate. The virus affects most of the internal organs of the animal and, in the acute phase of the disease, is invariably fatal with a mortality rate of nearly 100% (Gaskin in "Current Veterinary Therapy VI. Small Animal Practice", R. W. Kirk, ed., W. B. Saunders Co. (1977), pgs 1305; 1308). Cases of FIP disease have been reported throughout the world. The virus itself is highly contagious, affecting kittens as well as adult cats of all ages.

The FIP virus has recently been identified as a Coronavirus by Horzinek and Osterhaus, *Arch. Virol.* 59:1 (1979), and is, thus, the first identified feline Coronavirus. Attempts to culture the FIP virus *in vitro* have, however, been unrewarding. Growth of the virus in cell cultures of the peritoneal exudate of infected kittens was reported by Pedersen, *Am. J. Vet. Res.* 37:567 (1976), but attempts to grow the FIP agent in primary and continuous cell line cultures were unsuccessful. Hoshino and Scott, *Cornell Veterinarian* 68:411 (1978), likewise reported unsuccessful attempts to isolate FIP virus *in vitro*. Those researchers also described the replication of FIP virus in organ cultures of feline small intestine. Propagation of FIP virus in suckling mouse brain was achieved by Osterhaus et al., *Zentralbl. Veterinarmed.* 25B(4):301 (1978). The mouse-adapted virus strain has been replicated in the brains of suckling rats and hamsters by Horzinek and Osterhaus, *supra.*

Although attempts vave been made to protect cats against FIP disease by administration of inactivated crude tissue vaccines, all such attempts have failed to protect the animals (Gaskin, *supra*). In addition, virulent FIP virus, administered as a homogenized liver suspension, was used to vaccinate swine in a cross-protection study between FIP virus and porcine transmissible gastroenteritis (TGE) virus carried out by Woods and Pedersen, *Veterinary Microbiology*, 4:11 (1979), with inconclusive results.

Methods are known for preparing viruses and virus vaccines for the protection of cats against virus diseases. For example, GB 1,471,262 discloses and claims a method for preparing an antigenic material by forming a culture comprising the feline panleucopenia or mink enteritis virus, a nutrient medium and a cell strain derived from mink tissue containing actively dividing cells, growing the culture and either inactivating the virus, or attenuating the virus by serial passage of the cell strain. GB 1,492,930 discloses and claims a method of preparing an antigenic material effective against a virus disease in cats which comprises obtaining feline lung tissue free from virus disease, preparing a cell strain of the feline lung tissue, forming a culture of the cell strain, a tissue free virus corresponding to the virus disease and a nutrient medium, growing the culture and (a) inactivating the virus or attenuating the virus by serial passage.

It will be noted that in both of the above processes the virus material is first propagated and then either inactivated or attenuated by further serial passaging.

More recently, it has been reported that feline infectious peritonitis vaccine can be prepared by processes that are generally similar to those described above. For example, EP—0011864 discloses and claims a method of attenuating infectious peritonitis virus wherein an isolate of virulent infectious peritonitis virus is serially passaged in feline embryonic cell cultures about 100 times and the virus so attenuated is optionally propagated in susceptible cell lines to provide enough virus for a vaccine.

EP—0011864 claims priority from GB 7846603 where it is said that in order to achieve attenuation, above at least 100 passages are required.

The principle underlying the present invention is fundamentally different in concept from that in the patent specifications referred to above. In the known processes the vaccine is prepared by either attenuation or inactivation of the virus. Here, the vaccine is prepared not by attenuation but by dilution of the virulent vaccine.

According to the present invention there is provided a live feline infectious peritonitis vaccine comprising a virulent feline infectious peritonitis virus which, in an undiluted state, would cause feline infectious peritonitis disease symptoms when administered orally, nasopharyngeally, intramuscularly, subcutaneously, intraperitoneally or intraveously to susceptible cats and a pharmaceutically acceptable carrier, the amount of virus being such that the vaccine is capable of inducing immunity in animals of the genus felis, family felidae against feline infectious peritonitis without serious side effects. Where the virus is propagated by serial passage of feline embryonic cells, these cells are passaged less than 100 times.

The virus vaccines of the present invention can be prepared by a process which comprises propagating a population of susceptible feline cells infected with feline infectious peritonitis virus at a physiological temperature, harvesting the resultant virus and mixing it with a pharmaceutically acceptable carrier.

FIP virus can be propagated in feline cells from any source. For example the virus can be propagated in spleen, mesenteric lymph node, endothelial and kidney cells. The virus replicates in cells prepared directly from a cat organ or tissue or in continuous feline cell lines.

An infected cell population can be prepared by removing tissue, a sample or a whole organ from an infected cat anad disaggregating it into single cells. The tissue can be disaggregated firstly by mechanical disruption, for example mincing, releasing the cells from the disrupted tissue using a cell release agent for example trypsin and then removing any gross tissue stroma debris and cell release agent.

The resultant cells can be cultured and sub-cultured by conventional methods.

Alternatively an infected cell population can be obtained by isolating cells from infected tissue as described above, and mixing them with uninfected feline cells. The mixture can then be cultured and sub-cultured by conventional methods. The virus is transmitted from infected to uninfected cells during culturing. The infected cells need not be of the same type as the uninfected cells with which they are mixed thus infected lymph node cells can be mixed with uninfected kidney cells for example from a continuous kidney cell line.

The infected cells can be mixed with various proportions of uninfected cells, but the proportion of infected cells should not normally be less than 1% of the total number of cells. Preferably the proportion is 50:50%.

The infected cell populations described above can be cultured in any growth medium normally used for culturing mammalian cells. Examples of such media include Eagles', BME, MEM, McCoy's and Hank's balanced salt solution plus lactalbumin. The medium can be supplemented by an antibiotic for example penicillin or streptomycin or an antifungal for example fungazone. The medium can also be supplemented with up to 15% foetal calf serum.

The cells can be cultured at physiological temperatures for example from about 30° to about 40°C, preferably at 34—37°C.

Preferably the virus is propagated in primary feline cells.

Preferably the cells are kidney cells.

The presence of infectious virus in the supernatant liquid of cell cultures described above can be demonstrated by any standard *in vitro* test, for example viral titration in cell culture, serum neutralization, fluorescent antibody test or enzyme labelled immunoabsorbent assay. The supernatant liquid is infectious when administered orally, nasopharyneally intramuscularly, subcutaneously intraperitoneally or intravenously to susceptible cats.

After ascertaining that virus has multiplied in the cell culture the virus can be harvested mechanically by rupturing the cells for example by freezing and thawing to release the virus into the supernatant liquid culture medium. The supernatant liquid can be passed to other cell cultures.

Preferably the virus is passaged at least once in a stable feline cell line. More preferably the virus is passaged up to 30 or up to 100 times in such a cell line. Preferably the cell line is a kidney cell line.

The virus can be passaged up to at least 80 times without exhibiting any changes in virulence or morphology.

The virus content of the virus suspensions obtained on each passage increases in concentration to a maximum.

This virus content can be determined by a standard assay previously described.

When a suitable quantity of virus has been propagated the virus can be harvested mechanically as previously described and a cell free virus suspension can be obtained by removing any cells or cell debris. Virus particles can be isolated from these cell free suspensions by sedimentation through a sucrose-gradient.

The vaccine is then prepared according to standard pharmaceutical procedure by mixing a virus suspension with a carrier. Where the vaccine is intended for intra-ocular, oral or intranasal administration the carrier is a liquid for example an aqueous solution containing nutrients and/or stabilizers, for example Hank's balanced salt solution. Alternatively where the vaccine is for administration by injection the carrier is sterile pyrogen-free water for injection.

Preferably the vaccine is suitable for oral intra-ocular or intranasal administration.

Preferably the vaccine contains $10^2$ to $10^4$ $TCID_{50}$ $ml^{-1}$ of FIP virus ($TCID_{50}$ is the tissue culture infective dose which produces a cytopathic effect in 50% of the culture cells exposed to virus).

The vaccine of this invention can also contain other antigenic material derived from microorganisms which cause disease in members of the genus *Felix*. Examples of such materials include parvovirus (particularly feline panleucopenia), feline calicivirus, feline rhinotracheitis virus, feline rabies, feline retrovirus (particularly feline leukaemia) and feline herpes.

A polyvalent vaccine as described above comprises about 10 to 20% by volume of FIP virus. An example of one such vaccine comprises 10 to 20% by volume of FIP virus, 20 to 30% of calicivirus and 10 to 20% by volume of panleukapenia virus.

As is common practice the vaccines of this invention can be pregnated at a concentration appropriate for administration directly or a concentrate (for example a freeze dried solid) in a sealed container for example an ampoule which is dispensed in a package containing a second sealed container containing the carrier (for example sterile water) and instructions for reconstituting the vaccine.

Examples
Example A

The spleen of a cat which had died of an FIP infection was removed aseptically, minced into pieces approximately 4 to 5 mm square, washed three times with Hank's balanced salt solution and placed in a 1000 ml trypsinizing flask. Aqueous trypsin solution (0.25% in Hank's balanced salt solution; 200 ml) was added, the mixture was stirred using a teflon coated magnet and magnetic stirrer for 15 min, allowed to settle and the supernatant trypsin solution was decanted and discarded. A second portion of aqueous trypsin solution (200 ml) was added to the flask and the mixture was stirred for one hour. The cell-containing supernatant liquid was decanted under aseptic conditions and stored at 4°C.

The trypsin treatment was repeated until all the cells were removed from the tissue fragments and only organ stroma remained (four times).

The cooled decanted supernatant liquids were combined, filtered through sterile gauze and sedimented at 600 rpm (approximately 600×gravity) in a refrigerated Lourdes centrifuge. In order to free the cells from trypsin the cell pack was washed with Hank's balanced salt solution and resedimented. The procedure was repeated three times to eliminate residual trypsin.

The packed cells were diluted 1 to 100 with growth medium consisting of McCoy's medium supplemented with 10% foetal calf serum, penicillin (100 units ml⁻¹), streptomycin (500 ug ml⁻¹) and fungazone (10 units ml⁻¹).

One portion of cell suspension (100 ml) was placed into a one litre plastic Corning bottle and other portions (5 ml each) were placed into 60 mm Petri dishes containing 5 mm×16 mm glass cover slips. The Corning bottle was incubated at 37°C in an ordinary incubator and the Petri dishes were incubated at 37°C in a humidified carbon-dioxide incubator.

Within 24 hr, cells attached to the inside surface of the bottle and Petri dishes. Multiplication of the cells was allowed to proceed until a monolayer of cells covered the exposed surfaces under the medium. Versine and trypsin were added to the bottle to detach the cells. The detached cells were suspended in growth medium (200 ml) and replanted into two one litre plastic Corning bottles and 60 mm Petri dishes containing glass cover slips.

The cell monolayer which had formed on the cover slips in one of the original petri dishes was stained with hematolyn and eosin histostains and observed on a Leitz microscope. Multinucleated cells containing as many as 10 to 20 nuclei were noted in the stained cell preparations. Production of such multinucleated or giant cells (probably formed by fusion of infected cells) is evidence of virus infection and is a characteristic of well established coronaviridae.

The second passage of infected spleen cells produced abundant multinucleated cells on the glass cover slips which could be seen without staining. A portion of supernatant growth medium was withdrawn from one bottle and administered to SPF (specific pathogen free) cats. The cats developed FIP symptoms and died.

A total of six serial culture passages were carried out with FIP infected cells. A portion of supernatant growth medium was withdrawn after each passage and administered to an SPF cat. All the cats developed FIP symptoms.

Example B

An SPF cat was infected by orally administration of supernatant nutrient medium described in Example 1. When the cat was *in extremis* from the infection it was killed and the mesenteric lymph nodes were removed aseptically.

The isolated lymph nodes were diced into approximately 1 mm cubes, washed three times with Hank's balanced salt solution and mixed with aqueous trypsin solution (0.25% in Hank's balanced salt solution; 200 ml) in a one litre trypsinizing flask and the mixture was stirred for one hour using a teflon coated magnet and magnetic stirrer. The supernatant liquid containing suspended cells was decanted and stored at 4°C.

The trypsin treatment was repeated until nearly all the cells were in suspension and only tissue stroma remained (three times).

The trypsin-cell suspensions were combined and sedimented at 600 rpm for 10 min in a refrigerated Lourdes centrifuge. The resultant cell pack was resuspended in Hank's balanced salt solution and resedimented as previously described. The procedure was repeated three times to remove residual traces of trypsin. The cells thus obtained consisted of numerous types for example hematopoietic and epithelial cells and some were unidentified.

The cells were suspended in Eagle's MEM (minimum essential medium) containing 10% foetal calf serum and standard tissue culture antibiotics and antifungals (viz penicillin, streptomycin and fungizone) and placed in a one litre plastic screw cap Corning bottle. A quantity of feline kidney cells from a continuous feline kidney cell line (NL-FK-1) sufficient to grow to a monolayer in the Corning bottle were added and after withdrawing an aliquot (5 ml) the mixture was incubated in a standard incubator until a monolayer of cells could be observed microscopically.

The aliquot was used to inoculate a 60 mm Petri dish containing a glass cover slip. The inoculated dish was incubated in a humidified incubator in the presence of 5% carbon dioxide and 95% air.

When a monolayer of cells was observed (within 48 hr following infection) the glass cover slip was removed and stained with hematoxylineosin. Cytopathology indicating Coronaviridae infection was observed, that is to say formation of giant multinucleated cells and degeneration with retraction and detachment of the cell monolayer from the glass surface.

Supernatant liquid from the Corning bottle was removed and introduced into uninfected NL-FK-1 cells. The cells were incubated at 37°C until multinucleation could be observed in cells without staining.

Serial cultivation of FIP virus was carried out by repeated passaging more than 30 times in NL-FK-1 cells. Portions of supernatant liquid from passges 1 to 30 caused typical FIP clinical symptoms and death when administered orally, intraperitoneally, intravenously, subcutaneously or intramuscularly to susceptible cats. The virus also caused a cytopathic change in the NL-FK-1 cell line in resulting in the destruction of the cell monolayer.

The virus can be serially passed continuously and indefinely in the NL-FK-1 cell line.

FIP virus can be propagated by co-cultivating infected feline cells from other sources with other non-infected cells.

Example C

Supernatant liquid from an infected cell culture was sedimented at 25,000×gravity for 2 hr. The resultant pellet of virus was sedimented through a sucrose gradient the product stained with phosphotungstic acid and examined in a transmission electron microscope. The aggregate virus particles have a morphology characteristic of a Coronavirus. Its appearance resembles Avian Infectious Bronchitis virus the architypical Coronavirus. The FIP virus isolated was deposited in the American Type Culture collection on 21 August 1979 in accordance with Rule 28 of the European Patent Convention and has accession number VR-2004.

Example 1

A live virus vaccine was prepared by diluting 1 to 100 a cell free FIP virus suspension containing the maximum concentration of virus obtainable on repeated passaging. A dose (1.0 ml) was administered orally to two susceptible cats. Three weeks later a 1 to 50 dilution of the same material was administered to the same cat in an identical manner. No disease symptoms were observed in either of the treated animals.

Further dilution of FIP virus were made with increasing concentration from 1 to 50 to 1 to 5 in ascending steps. At the end of successive three weeks periods each cat was given a dose (1.0 ml) of the next most concentrated vaccine. Three weeks after administration of the final dilution, the cats were challenged with 1.0 ml of undiluted FIP infected tissue suspension by the oral route. Two susceptible animals were similarly infected at the same time. The unvaccinated animals exhibited classic FIP disease symptoms and died at seven and ten days post infection. The vaccinated animals remained healthy and free of any symptoms of disease.

After two weeks, the immunity of the two surviving vaccinated animals was again challenged. Two control unvaccinated cats succumbed to the disease, while the immunized animals remained healthy and free of disease symptoms. A third challenge three weeks after the second challenge and a fourth challenge six months after the third resulted in the vaccinated cats remaining healthy and free of disease symptoms and all control unvaccinated animals (two per challenge) succumbing to FIP disease.

**Claims for the Contracting States BE, CH, DE, FR, GB, LI, LU, NL, SE:**

1. A live feline infectious peritonitis vaccine comprising a virulent feline infectious peritonitis virus which, in an undiluted state, would cause feline infectious peritonitis disease symptoms when administered orally, nasopharylgeally, intramuscularly, subcutaneously, interperitoneally or intravenously to susceptible cats and a pharmaceutically acceptable carrier, the amount of virus being such that the vaccine is capable of inducing immunity in animals of the genus felis, family felidae against feline infectious peritonitis without serious side effects provided that where the virus is propagated by serial passage of feline embryonic cells, these cells are passaged less than 100 times.

2. A vaccine as claimed in claim 1 where the virus is ATCC No. VR-2004.

3. A vaccine as claimed in claim 1 or claim 2 where the virus is present in an amount of from $10^2$ to $10^4$ $TCID_{50}$/ml.

4. A vaccine as claimed in any one of claims 1 to 3 further comprising second antigenic material.

5. A process for preparing a vaccine as claimed in claim 1 which comprises propagating susceptible feline cells infected with feline infectious peritonitis virus (and where such propagation involves serial passage in feline embryonic cells, cells are passaged less than 100 times) at a physiological temperature, without causing substantial attenuation of the virulence of the virus, harvesting the resulting viral material and mixing it with a pharmaceutically acceptable carrier.

6. A process as claimed in claim 5 where the feline cells are primary cells.

7. A process as claimed in claim 6 where the cells are kidney cells.

8. A process as claimed in any one of claims 5 to 7 where the virus is passaged at least once in feline kidney cells.

9. A process as claimed in claim 8 where the virus is passaged up to 30 times.

10. A process as claimed in claim 8 where the virus is passaged up to 80 times.

11. A process as claimed in any one of claims 5 to 10 where the cell line is infected by co-cultivating uninfected cells with infected cells.

12. A process as claimed in claim 11 where the infected cells are lymph node cells.

**Claim for the Contracting State AT:**

A process for preparing a live feline infectious peritonitis vaccine comprising a virulent feline infectious peritonitis virus which, in an undiluted state, would cause feline infectious peritonitis disease symptoms when administered orally, nasopharyngeally, intramuscularly, subcutaneously, intraperitoneally or intravenously to susceptible cats and a pharmaceutically acceptable carrier, the amount of virus being such that the vaccine is capable of inducing immunity in animals of the genus felis, family felidae against feline infectious peritonitis without serious side effects which comprises propagating susceptible feline cells infected with feline infectious peritonitis virus (and where such propagation involves serial passage in feline embryonic cells, cells are passaged less than 100 times) at a physiological temperature, without causing substantial attenuation of the virulence of the virus, harvesting the resulting viral material and mixing it with a pharmaceutically acceptable carrier.

### Patentansprüche für die Vertragsstaaten BE, CH, DE, FR, GB, LI, LU, NE, SE:

1. Lebendimpfstoff gegen infektiöse Peritoneitis von Katzen, enthaltend ein virulentes, infektiöse Peritoneitis bei Katzen erregendes Virus, das in Verbindung mit einem pharmazeutisch verträglichen Träger bei oraler, nasopharyngealer, intramuskulärer, subkutaner, intraperitonealer oder intravenöser Verabfolgung an suszeptible Katzen in unverdünnter Form die Krankeitssymptome der infektiösen Peritoneitis von Katzen hervorrufen würde, in einer Virusmenge, bei der der Impfstoff Tiere der Gattung Felis, Familie Felidae, ohne beträchtliche Nebenwirkungen gegen infektiöse Peritoneitis von Katzen immunisiert, vorausgesetzt daß, wenn das Virus durch eine Reihe von Passagen embryonaler Katzenzellen vermehrt wird, diese Zellen weniger als 100 mal passagiert werden.

2. Impfstoff nach Anspruch 1, enthaltend das Virus ATCC VR-2004.

3. Impfstoff nach Anspruch 1 oder 2, dadurch gekennzeichnet, daß das Virus in einer Menge von $10^2$ bis $10^4$ $TCID_{50}$/ml enthalten ist.

4. Impfstoff nach einem der Ansprüche 1 bis 3, dadurch gekennzeichnet, daß eine zweite antigene Substanz enthalten ist.

5. Verfahren zur Herstellung eines Impfstoffs nach Anspruch 1, dadurch gekennzeichnet, daß man mit dem bei Katzen infektiöse Peritoneitis erregenden Virus infizierte suszeptible Katzenzellen bei einer physiologischen Temperatur vermehrt (und wenn die Vermehrung eine Reihe von Passagen in embryonalen Katzenzellen beinhaltet, die Zellen weniger als 100 Mal passagiert), ohne dabei eine wesentliche Attenuierung der Virulenz des Virus zu verursachen, das gebildete Virusmaterial erntet und es mit einem pharmazeutisch verträglichen Träger mischt.

6. Verfahren nach Anspruch 5, in dem die Katzenzellen Primärzellen sind.

7. Verfahren nach Anspruch 6, in dem die Zellen Nierenzellen sind.

8. Verfahren nach einem der Ansprüche 5 bis 7, dadurch gekennzeichnet, daß man das Virus mindestens einmal in Katzen-Nierenzellen passagiert.

9. Verfahren nach Anspruch 8, dadurch gekennzeichnet, daß das Virus bis zu 30 Mal passagiert wird.

10. Verfahren nach Anspruch 8, dadurch gekennzeichnet, daß das Virus bis zu 80 Mal passagiert wird.

11. Verfahren nach einem der Ansprüche 5 bis 10, dadurch gekennzeichnet, daß die Zellinie durch gemeinsame Kultivierung nicht-infizierter Zellen mit infizierten Zellen infiziert wird.

12. Verfahren nach Anspruch 11, dadurch gekennzeichnet, daß die infizierten Zellen Lymphknotenzellen sind.

### Patentanspruch für den Vertragsstaat AT:

Verfahren zur Herstellung eines Lebendimpfstoffs gegen infektiöse Peritoneitis von Katzen, enthaltend ein virulentes, infektiöse Peritoneitis bei Katzen erregendes Virus, das in Verbindung mit einem pharmazeutisch verträglichen Träger bei oraler, nasopharyngealer, intramuskulärer, subkutaner, interperitonealer oder intravenöser Verabfolgung an suszeptible Katzen in unverdünnter Form die Krankheitssymptome der infektiösen Peritoneitis von Katzen hervorrufen würde, in einer Virusmenge, bei der der Impfstoff Tiere der Gattung Felis, Familie Felidae, ohne beträchtliche Nebenwirkung gegen infektiöse Peritoneitis von Katzen immunisiert, dadurch gekennzeichnet, daß man mit dem bei Katzen infektiöse Peritoneitis erregenden Virus infizierte suszeptible Katzenzellen bei einer physiologischen Temperatur vermehrt (und wenn die Vermehrung eine Reihe von Passagen in embryonalen Katzenzellen beinhaltet, die Zellen weniger als 100 mal passagiert), ohne dabei eine wesentliche Attenuierung der Virulenz des Virus zu verursachen, das gebildete Virusmaterial erntet und es mit einem pharmazeutisch verträglichen Träger mischt.

### Revendications pour les Etats Contractants BE, CH, DE, FR, GB, LI, LU, NL, SE:

1. Un vaccin vivant de la péritonite infectieuse des félins comprenant un virus virulent de la péritonite infectieuse des félins, qui, à l'état non dilué, provoquerait les symptômes de la maladie de la péritonite infectieuse des félins, une fois adminitré par voie orale, nasopharingienne, intramusculaire, sous-cutanée, intra-péritonéale ou intra-veineuse à des chats susceptibles et un support pharmaceutiquement aceptable, la quantité de virus étant telle que le vaccin soit capable d'induire chez les animaux du genre Félix, famille des félides l'immunité contre la péritonite infectieuse des félins, sans effets secondaires graves, pourvu que lorsque le virus est propagé par des passages en série dans les cellules embryonnaires de félins, ces cellules soient passées moins de 100 fois.

2. Un vaccin comme revendique à la revendication 1 dans lequel le virus est ATCC No. VR-2004.

3. Un vaccin comme revendiqué à la revendication 1 ou 2 dans lequel le virus est présent en une quantité allant de $10^2$ à $10^4$ $TCID_{50}$/ml.

4. Un vaccin comme revendiqué dans n'importe laquelle des revendications de 1 à 3 et comprenant en outre une autre substance antigénique.

5. Un procédé pour la préparation d'un vaccin commen revendiqué à la revendication 1 qui comprend la propagation de cellules de félins susceptibles infectées par le virus de la péritonite infectieuse des félins (et dans les cas où une telle propagation comprend les passages en série dans les cellules embryonnaires des félins, les cellules sont passées moins de 100 fois) à une température physiologique, sans provoquer d'atténuation notoire de la virulence du virus, en récoltant la matière virale en résultant et en la mélangeant avec un support pharmaceutiquement acceptable.

6. Un procédé, comme revendiqué à la revendication 5 dans laquelle les cellules de félins sont des cellules primaires.

7. Un procédé comme revendiqué à la revendication 6 dans laquelle les cellules sont des cellules de reins.

8. Un procédé comme revendiqué aux revendications de 5 à 7 dans lesquelles le virus passe au moins une fois dans les cellules de reins de félins.

9. Un procédé comme revendiqué à la revendication 8 dans laquelle le virus passage jusqu'à 30 fois.

10. Un procédé comme revendiqué à la revendication 8 dans laquelle le virus passe jusqu'à 80 fois.

11. Un procédé comme revendiqué dans n'importe laquelle des revendications de 5 à 10 dans laquelle la ligne de cellules est infectée en mettant en culture ensemble les cellules non infectées et les cellules infectées.

12. Un procédé comme revendiqué à la revendication 11 dans laquelle les cellules infectées sont les cellules des ganglions lymphatiques.

**Revendication pour l'Etat Contractant AT:**

Un procédé pour le préparation d'un vaccin vivant de la péritonite infectieuse des félins, comprenant un virus virulent de la péritonite infectieuse des félins, qui, dans un état non dilué, peut provoquer les symptômes de la maladie de la péritonite infectieuse s'il est administré par voie orale, nasopharingienne, intramusculaire, sous-cutanée, intra-péritonéale ou intraveineuse à des chats susceptibles et un support pharmaceutiquement acceptable, la quantité du virus étant telle que le vaccin soit capable d'induire chez les animaux du genre Félix, famille des félides, l'immunité contre la péritonite infectieuse des félins sans effets secondaites graves, et qui comprend la propagation de cellules de félins susceptibles infectées du virus de la péritonite infectieuse des félins (et lorsqu'une telle propagation comprend le passage en série dans les cellulse embryonnaires de félins, les cellules étant traversées pas moins que 100 fois) à une température physiologique, sans provoquer d'atténuation notoire de la virulence du virus, le récolte de la matière virale en résultant et le mélange de la même avec une support pharmaceutiquement acceptable.